Europäisches Patentamt

European Patent Office

Office européen des brevets

(19)

(11) **EP 1 163 927 B1**

(12) **EUROPEAN PATENT SPECIFICATION**

(45) Date of publication and mention
of the grant of the patent:
**05.01.2005  Bulletin 2005/01**

(51) Int Cl.⁷: **A61N 1/36**, A61N 1/32

(21) Application number: **01114513.3**

(22) Date of filing: **15.06.2001**

(54) **Apparatus for electrostimulation of the human body**

Vorrichtung zur Elektrostimulation des menschlichen Körpers

Appareil de stimulation électrique du corps humain

(84) Designated Contracting States:
**AT BE CH CY DE DK ES FI FR GB GR IE IT LI LU
MC NL PT SE TR**

(30) Priority: **16.06.2000  IT  BO000360**

(43) Date of publication of application:
**19.12.2001  Bulletin 2001/51**

(73) Proprietor: **GROUP TALAMONTI DI TALAMONTI
ALESSANDRO DITTA INDIVIDUALE
63039 San Benedetto del Tronto (IT)**

(72) Inventor: **Talamonti, Alessandro
63035 Offida (IT)**

(74) Representative: **Bongiovanni, Simone et al
Studio Torta S.r.l
Via Viotti, 9
10121 Torino (IT)**

(56) References cited:
**WO-A-00/15295          WO-A-99/06106
FR-A- 2 617 722          US-A- 5 285 781
US-A- 5 817 138**

## Description

Apparatuses are known which, via application of pulses, for example of an electrical nature, to the human body are able to increase the muscular mass, improve muscular elasticity, increase aerobic resistance, increase resistance to fatigue, develop the capillary flow, or else simply perform a relaxing massage on parts of the body that are aching.

The present invention falls within the above sphere, but relates, in particular, to an electrostimulation apparatus for the generation of electrical signals designed to activate the molecules constituting the lymph that circulates in the human body.

The apparatus that forms the subject of the present invention is based on the physical principle of sound resonance applied to microtargets, such as amino acids or small protein aggregates, which, since they possess a particular wavelength of their own, enter into resonance with the incident wave, provided that the latter, of course, possesses a frequency which is equal or similar to the frequencies of the microtargets.

In fact, document US-A-5 817 138 discloses an apparatus for electrostimulation of the human body by means of treatment waves obtained by superimposing, on a first wave having a first frequency, a second wave having a second frequency. The second frequency is higher than the first frequency.

Furthermore, from US-A-5 285 781 it's well known the use of program cards to program the stimulator and allows for closed loop feedback.

In addition to the frequency, it is very important that the waves used for the treatment should have a low voltage and possess a particular form, as will be specified better later. Consequently, the present apparatus differs markedly, from numerous standpoints, from similar electrotherapy apparatuses present on the market, above all for the fact that it uses particularly low voltages, of between, for example $\pm 7$ and $\pm 20$ V, with particular waveforms. In this connection, it is recalled that traditional apparatuses use much higher voltages, for example $\pm 40$ V.

According to the present invention, an apparatus is provided for electrostimulation of the human body, as described and claimed in Claim 1.

The present invention will now be described with reference to the attached drawings, which are provided purely to furnish a non-limiting example of embodiment, and in which:

- Figure 1 is a simplified diagram of the apparatus that forms the subject of the present invention;
- Figure 2 represents a first carrier wave having a first frequency;
- Figure 3 illustrates a second wave that is superimposed on the carrier wave and has a higher frequency than the carrier wave;
- Figure 4 is a block diagram of a typical treatment that may be carried out using the apparatus of Figure 1.

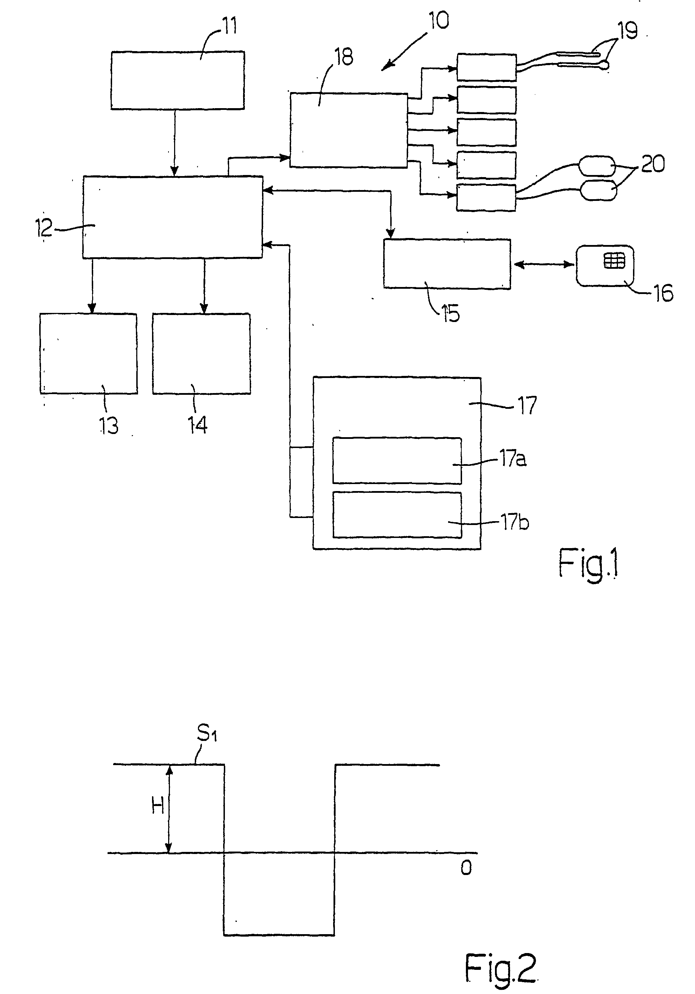

In Figure 1, the reference number 10 designates a simplified diagram of the apparatus that forms the subject of the present invention.

In the apparatus 10 it is possible to distinguish a control keypad 11 which represents the main interface device between the user and the apparatus 10 itself.

By means of the keypad 11, the user can choose one of more programmes, each of which can be designated, for example, by a corresponding letter of the alphabet T-G-L-Y-O-R-S-M-P-V-I-B.

The keypad 11 can be employed by the user also for selecting, for instance, the starting point and the finishing point of the treatment to be performed, the level of intensity of the current to be sent to the electrodes, the duration of the treatment itself, and possibly the intensity of acoustic emission (see later).

All the operations are controlled by a microprocessor 12 which can be electronically accessed through the aforesaid control keypad 11.

The microprocessor 12 is designed to control all the operations that will be described in what follows.

As shown again in Figure 1, to the microprocessor 12 is connected a display device 13 by means of which the user can visually monitor all the operating steps of the apparatus 10. In particular, the device 13 may comprise a plurality of LEDs, or else may comprise a liquid-crystal display.

In addition, if he so wishes, the user can also activate a function of acoustic reproduction of the waveforms of the treatment in progress by means of an acoustic-signalling module 14, which is also electronically connected to the microprocessor 12.

The apparatus 10 further comprises a non-volatile memory 15 containing the data on the individual waveforms of each treatment and the parameters for execution of each treatment. It is possible to modify these parameters by reprogramming the memory 15, or else by using an electronic card 16 to be inserted into the apparatus 10, there also existing the possibility for the said electronic card 16 being programmed and personalized for each user.

To increase safety of the entire system, the apparatus 10 also comprises a safety device 17 in which it is possible to distinguish a sensor 17a for measuring arterial pressure before, during and after a given treatment. Should the reading of the patient's arterial pressure be outside the tolerance limits allowed, this occurrence is stored, and the apparatus 10 signals it to the user, for example by means of the display device 13; at the same time, the apparatus 10 interrupts the treatment in progress.

The safety device 17 moreover comprises a sensor 17b designed to detect the impedance of the patient's body and to compare it with values that are considered normal. Also in this case, if the apparatus 10 finds that the values measured do not fall within the norm, it interrupts the treatment in progress.

The microprocessor 12 also controls a module 18 that generates the treatment waves. The said waves are sent to the outputs, and the electrical stimuli are applied to the patient's body using pads 19 and/or conductive plates 20. Present on each output is a feedback control of the energy supplied.

In an embodiment of the present invention, each output has the same value as the stimulus and the same waveform, but it is possible to envisage other embodiments with outputs having different values and possibly with different waveforms, in such a way as to generate simultaneously two different forms of treatment in different parts of the patient's body.

The principle on which the generation of treatment waves is based is that of acoustic resonance.

In this way, it has been possible to obtain activation of the molecules constituting the lymph and their migration along the physiological channels that present low resistance, thus achieving a physiological drainage.

In particular, if application of the pads 19 and/or electrodes 20 is in the classic points indicated by Chinese acupuncture, a sort of molecular agitation of the extracellular liquids and of the albumin molecules present in the said points is brought about. This agitation which, as has been said, takes place via the topical application of the waves generated by the apparatus 10, results, in short, in a centripetal solicitation of the molecules making up the extracellular liquids, which are conveyed through the main and secondary lymphatic networks to the lymph-node stations and the central venous system.

The waves produced by the apparatus 10 may also act, in a traditional way, on the muscular system and on the regional lipid metabolism, with morphological modifications of the subcutaneous fatty tissue and with reduction in the mass of the latter.

In order to establish the type of wave that is to be generated by the module 18, a number of hypotheses have been formulated:

- the behaviour of the target, for example an amino acid or a small protein aggregate, to be attacked by means of the wave train may be likened to that of a system designed to oscillate, hence one provided with natural frequencies of oscillation (eigenfrequencies);
- a single longitudinal wave incident on the target is considered; and finally
- the absence is assumed of interaction between the target and the other constituents of the cell structure.

It is known that, whenever an oscillating system is subjected to a periodic series of pulses, in the case in point in the form of waves, having a frequency comparable to one of the natural frequencies of oscillation of the system, the latter will oscillate with a progressively increasing amplitude.

Suppose that the force applied to the target, for example an amino acid, is given by the formula

$$F = F_o \cos \omega t$$

where $F_0$ is the maximum value of the force, $t$ is the time, and $\omega$ is the angular frequency.

Suppose moreover that the target is also subjected to an elastic force $- k \, x$ linked to its vibration about the position of equilibrium, and to a damping $- d \, v$ due to the friction that acts on the target.

Evidently, in a traditional manner, the oscillation elastic constant is designated by $k$, whilst $d$ represents the constant of proportionality between the force of friction and the speed $v$ at which the target oscillates.

The equation of the motion of the target will thus be

$$m \, a = - k \, x - d \, v + F_o \cos \omega t$$

where $m$ is the mass of the target and $a$ is the acceleration.

From the resulting differential equation we have that the target is forced to oscillate at the angular frequency $\omega$ of the force F applied. Consequently, the displacements of the target are given by

$$x = A \sin (\omega t - \varphi)$$

where $\varphi$ indicates the initial step, whilst $A$ indicates the maximum amplitude of the wave.

$$A = \frac{F_0/m}{\sqrt{\left(\omega^2 - \omega_0^2\right)^2 + (d/m)^2 \, \omega^2}}$$

$$\tan g\varphi = \frac{\omega^2 - \omega_0^2}{d\omega/m}$$

whilst

$$\omega_0^2 = k/m$$

The form $x = A \sin (\omega \, t - \varphi)$ would appear to indicate that the forced oscillations do not undergo damping but have a constant amplitude and a frequency equal to that of the applied force when the latter predominates over the damping forces.

Obviously, the smaller the damping, the greater the resonance; assuming that it were possible to have a constant of friction $d = 0$, the amplitude of the wave $A$ would become infinite for $\omega_0 = \omega$.

In this case, the kinetic energy of the target would be maximum and there would be the so-called energization of the structure.

For $\varphi = 0$, since the foregoing equation expresses the position of the target, it would become:

$$x = A \sin \omega t$$

The speed of the target is of course obtained by differentiating the above expression, so that

$$v = A \, \omega \cos \omega t$$

The increase in temperature found on the target may be explained by the braking action exerted by the tissues in regard to the incident wave having a high energy transport.

The said energy is proportional to the square of the oscillation frequency $f$, to the velocity $v$ and to the square of the amplitude $A$.

The heat that is produced in the structure is due to the non-perfect elasticity of the target when the latter is traversed by the wave.

To cause the target - for example, molecules making up the liquid of the extracellular sector - to enter into resonance, it has been found experimentally, also on the basis of prior theoretical considerations, that, as illustrated in Figure 2, the wave generated by the module 18 must comprise a biphasic carrier wave $S_1$ compensated at a low voltage, the amplitude of which is preferably but not necessarily between -12V and +12V. In actual fact, the carrier wave $S_1$ for inducing similar effects on tissues could have a voltage of between $\pm 7V$ and $\pm 20V$.

Preferably, the carrier wave $S_1$ is applied alternately in the positive half-plane (0V and +12V) and in the negative half-plane (-12V and 0V).
In this way, the tissues being treated do not undergo any polarization in so far as the electrical charge transferred is practically zero.

In fact, the carrier wave $S_1$, having an amplitude H, is transferred by means of pairs of pads 19 and/or plates 20 with positive/negative polarization induced alternately by applying packets or trains of signals in the positive half-plane or negative half-plane for alternated and equal time intervals.

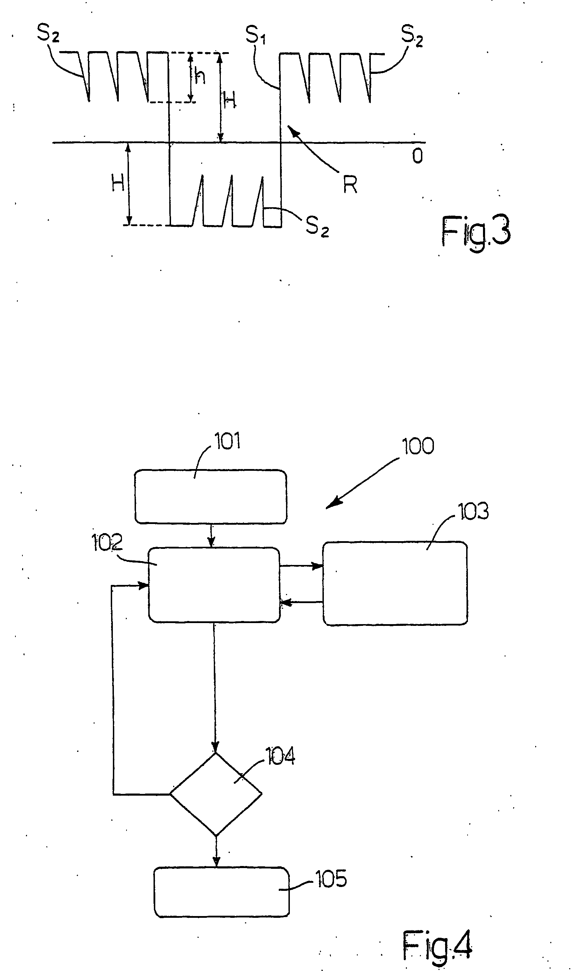

As shown in Figure 3, superimposed on the said carrier wave $S_1$ there must be a an elementary wave, for instance, a sawtooth wave $S_2$ (Figure 3) having a frequency of between 300 Hz and 2300 Hz. The wave $S_2$ is modulated by the wave $S_1$ which has a frequency of between 0.3 Hz and 7 Hz.

In this connection, it is to be noted that the waves generated in the apparatuses of the prior art normally have frequencies of between 1 Hz and 120 Hz.

In other words, the wave system that hits the target is made up of the combination of a modulating and carrier wave $S_1$ having an amplitude h and a sawtooth wave $S_2$ to give rise to a resultant wave R.

Finally, we shall have the following optimal values:

T = 70 msec/1.4 sec with frequencies of between 0.33 Hz and 7 Hz for the wave $S_1$; and
P = 0.475 msec/2.5 msec with frequencies of between 300 Hz and 2300 Hz for the wave $S_2$.

Fibrillation of the target due to the impact with the incident wave leads to the following effects:

- an increase in local-regional temperature;
- a translation of the excited molecules along the lymphatic pathways in a passive way;
- an activation of the drainage systems in the sectors concerned;
- an action of removal of the plasmatic components pathologically present in the extracellular spaces, with corresponding modification of the ratios of local onco/osmosis.

Figure 4 shows a block diagram 100 of a typical treatment.

If the number 101 designates the treatment operation of choice performed by the operator in 102, the microprocessor 12 controls proper generation of the wave R and at the same time verifies that the pre-set level of intensity is as independent as possible of the characteristics of the body on which the pads 19 and/or plates 20 are applied. In fact, it is known that the waveform is deformed according to the load to which it is applied, hence supplying a quantity of energy that depends on the load itself. The electrical pulses applied to the human body do not escape this general rule. However, the adaptive control obtained by means of the apparatus 10 guarantees that the energy transferred is equal to the energy pre-set by the user, in so far as the system continuously makes a comparison between the energy transferred and the energy set, automatically adapting the output level.

The aforesaid adaptive control is carried out in the step 103 illustrated in Figure 4, whilst in 104 the microprocessor 12 decides whether the treatment is terminated or not. In the event of an affirmative answer, the system stores the data and stops operation of the apparatus 10, as envisaged in step 105, whereas in the case where the system decides that treatment should be continued, it goes back to step 102, and so forth until the treatment envisaged is completed.

By means of the apparatus 10 it is possible to implement automatic work sequences in which a certain number of treatments are carried out consecutively and automatically.

For instance, it is possible to implement a sequence made up of eight consecutive treatments, each having a duration of one minute. This sequence is initialized starting from step 101 of Figure 4 and, for example, from the treatment designated by the letter P.

## Claims

1.  An apparatus (10) for electrostimulation of the human body, comprising a control keypad (11), a microprocessor (12) designed to receive commands from said keypad (11), a display device (13), a memory (15) containing the data on the individual waveforms of each treatment and the parameters of execution of the treatment, a module (18) generating treatment waves, the module (18) being controlled by the microprocessor (12), and at least one pair of elements (19, 20) for applying the waves produced by said module (18) on the patient's body; wherein said module (18) is designed to generate a wave (R) obtained by superimposing, on a first wave (S1) having a first frequency, a second wave (S2) having a second frequency, said second frequency being higher than said first frequency; and wherein the apparatus (10) further comprises a sensor (17b) designed for detecting the impedance of the patient's body and for comparing it with values deemed normal; the apparatus (10) being **characterized in that** it is moreover provided a safety device (17) which in turn comprises a sensor (17a) for measuring the arterial pressure before, during and after execution of a given treatment.

2.  An apparatus (10) as claimed in Claim 1, in which the waves applied on the patient's body have a frequency such as to generate a resonance of the molecules making up the body.

3.  An apparatus (10) as claimed in Claim 2, in which said molecules constitute the lymph circulating in the human body.

4.  An apparatus (10) as claimed in Claim 3, in which said first wave (S1) possesses a frequency of between 0.33 Hz and 7 Hz, whilst said second wave (S2) possesses a frequency of between 300 Hz and 2300 Hz.

5.  An apparatus (10) as claimed in any of the foregoing claims, in which said first wave (S1) is a square wave, whilst said second wave (S2) is a sawtooth wave.

6.  An apparatus (10) as claimed in Claim 5, in which said first wave (S1) has a voltage of between ± 7V and ± 20V.

7.  An apparatus (10) as claimed in any of the foregoing claims, in which said memory (15) may be reprogrammed as required and personalized according to the user's data.

8.  An apparatus (10) as claimed in Claim 7, in which the reprogramming and personalization of said memory (15) is performed by entering an electronic card (16) in the apparatus (10).

9.  An apparatus (10) as claimed in any of the foregoing claims, in which there are moreover provided means for feedback control of the energy supplied to the patient's body.

## Patentansprüche

1.  Vorrichtung (10) zur Elektrostimulation des menschlichen Körpers, umfassend ein Steuerungstastenfeld (11), einen Mikroprozessor (12), welcher zur Aufnahme von Befehlen des Tastenfelds (11) eingerichtet ist, eine Anzeigevorrichtung (13), einen Speicher (15), welcher die Daten der individuellen Wellenformen jeder Behandlung und die Parameter zur Durchführung der Behandlung enthält, ein Modul (18), welches Behandlungswellen erzeugt, welches Modul (18) durch den Mikroprozessor (12) gesteuert wird, und wenigstens ein Paar von Elementen (19, 20), um die durch das Modul (18) erzeugten Wellen an dem Körper des Patienten anzuwenden; wobei das Modul (18) eingerichtet ist, um eine Welle (R) zu erzeugen, welche dadurch erhalten wird, indem einer ersten Welle (S1) mit einer ersten Frequenz eine zweite Welle (S2) mit einer zweiten Frequenz überlagert wird, welche zweite Frequenz höher ist als die erste Frequenz; und wobei die Vorrichtung (10) weiter einen Sensor (17b) umfasst, welcher zur Erfassung der Impedanz des Körpers des Patienten und um diese mit als normal angesehenen Werten zu vergleichen eingerichtet ist; welche Vorrichtung (10) **dadurch gekennzeichnet ist, dass** sie zudem mit einer Sicherheitsvorrichtung (17) versehen ist, welche wiederum einen Sensor (17a) umfasst, um den arteriellen Druck vor, während und nach der Ausführung einer bestimmten Behandlung zu messen.

2.  Vorrichtung (10) nach Anspruch 1, in welcher die auf den Körper des Patienten angewandten Wellen eine Frequenz haben, um eine Resonanz der den Körper bildenden Moleküle zu erzeugen.

3.  Vorrichtung (10) nach Anspruch 2, in welcher die Moleküle die in dem Körper zirkulierende Lymphe bilden.

4.  Vorrichtung (10) nach Anspruch 3, in welcher die erste Welle (S1) eine Frequenz zwischen 0,33 Hz und 7 Hz aufweist, während die zweite Welle (S2) eine Frequenz zwischen 300 Hz und 2300 Hz aufweist.

5. Vorrichtung (10) nach einem der vorhergehenden Ansprüche, in welcher die erste Welle (S1) eine Rechteckwelle ist, während die zweite Welle (S2) eine Sägezahnwelle ist.

6. Vorrichtung (10) nach Anspruch 5, in welcher die erste Welle (S1) eine Spannung zwischen ±7V und ±20V hat.

7. Vorrichtung (10) nach einem der vorhergehenden Ansprüche, in welcher der Speicher (15) wie benötigt umprogrammiert und entsprechend der Anwenderdaten personalisiert werden kann.

8. Vorrichtung (10) nach Anspruch 7, in welcher die Umprogrammierung und Personalisierung des Speichers (15) durchgeführt wird, indem eine elektronische Karte (16) in die Vorrichtung eingesetzt wird.

9. Vorrichtung (10) nach einem der vorhergehenden Ansprüche, in welcher zudem Mittel zur Feedbacksteuerung der dem Körper des Patienten zugeführten Energie vorgesehen sind.

**Revendications**

1. Appareil (10) de stimulation électrique du corps humain, comprenant un clavier de commande (11), un microprocesseur (12) conçu pour recevoir des commandes dudit clavier (11), un dispositif d'affichage (13), une mémoire (15) contenant les données sur les différentes formes d'onde de chaque traitement et les paramètres d'exécution du traitement, un module (18) générant des ondes de traitement, le module (18) étant commandé par le microprocesseur (12), et au moins une paire d'éléments (19, 20) servant à appliquer les ondes générées par ledit module (18) sur le corps du patient ; dans lequel ledit module (18) est conçu pour générer une onde (R) obtenue en superposant, sur une première onde (S1) présentant une première fréquence, une seconde onde (S2) présentant une seconde fréquence, ladite seconde fréquence étant plus élevée que ladite première fréquence ; et dans lequel l'appareil (10) comprend en outre un capteur (17b) conçu pour détecter l'impédance du corps du patient et servant à la comparer aux valeurs considérées comme normales ; l'appareil (10) étant **caractérisé en ce qu'**il est par ailleurs équipé d'un dispositif de sécurité (17) qui comprend à son tour un capteur (17a) pour mesurer la pression artérielle avant, pendant et après l'exécution d'un traitement donné.

2. Appareil (10) selon la revendication 1, dans lequel les ondes appliquées sur le corps du patient présentent une fréquence servant à générer une résonance des molécules constituant le corps.

3. Appareil (10) selon la revendication 2, dans lequel lesdites molécules constituent la lymphe circulant dans le corps humain.

4. Appareil (10) selon la revendication 3, dans lequel ladite première onde (S1) possède une fréquence comprise entre 0,33 Hz et 7 Hz, tandis que ladite seconde onde (S2) possède une fréquence comprise entre 300 Hz et 2300 Hz.

5. Appareil (10) selon l'une quelconque des revendications antérieures, dans lequel ladite première onde (S1) est une onde carrée, tandis que ladite seconde onde (S2) est une onde en dent de scie.

6. Appareil (10) selon la revendication 5, dans lequel ladite première onde (S1) présente une tension comprise entre ± 7 V et ± 20 V.

7. Appareil (10) selon l'une quelconque des revendications antérieures, dans lequel ladite mémoire (15) peut être reprogrammée de la façon requise et personnalisée selon les données de l'utilisateur.

8. Appareil (10) selon la revendication 7, dans lequel la reprogrammation et la personnalisation de ladite mémoire (15) sont effectuées en introduisant une carte électronique (16) dans l'appareil (10).

9. Appareil (10) selon l'une quelconque des revendications antérieures, dans lequel des moyens sont fournis pour réguler l'énergie fournie au corps du patient.

Fig.1

Fig.2

Fig.3

Fig.4